# DEMANDE DE BREVET EUROPEEN

(11) **EP 3 557 677 A1**
(43) Date de publication de la demande: **23.10.2019**
(21) Numéro de dépôt: 19170564.9
(22) Date de dépôt: 09.03.2015
(51) Int. Cl.: H01M 10/052, H01M 10/0568, H01M 10/0569, C07D 233/90

(54) **AMELIORATION DE LA CONDUCTIVITE IONIQUE D'ELECTROLYTE A BASE DE SELS DE LITHIUM D'IMIDAZOLATE**

(30) Priorité: 14.03.2014 FR 1452145; 30.05.2014 FR 1454902
(62) Demande divisionnaire de: 15713996.5
(71) Demandeur: Arkema France, 92700 Colombes (FR)
(72) Inventeur: SCHMIDT, Grégory, 69493 69493PIERRE-BENITE Cedex (FR)
(74) Mandataire: Chahine, Audrey Claire

(57) **Abrégé**

La présente invention concerne une composition comprenant au moins un électrolyte à base de sels d'imidazolate de lithium et l'utilisation de solvants nitriles ou dinitriles pour l'augmentation de la conductivité ionique d'électrolyte à base de sels d'imidazolate de lithium. Elle a également pour objet l'utilisation de la composition électrolytique dans les batteries Li-ion.

## Description

### DOMAINE DE L'INVENTION

La présente invention concerne une composition comprenant au moins un électrolyte à base de sels d'imidazolate de lithium et l'utilisation de solvants nitriles ou dinitriles pour l'augmentation de la conductivité ionique d'électrolyte à base de sels d'imidazolate de lithium. Elle a également pour objet l'utilisation de la composition électrolytique dans les batteries Li-ion.

### ARRIERE-PLAN TECHNIQUE

Une batterie lithium-ion comprend au moins une électrode négative (anode), une électrode positive (cathode), un séparateur et un électrolyte. L'électrolyte est constitué généralement d'un sel de lithium dissous dans un solvant qui est généralement un mélange de carbonates organiques, afin d'avoir un bon compromis entre la viscosité et la constante diélectrique. Des additifs peuvent ensuite être ajoutés pour améliorer la stabilité des sels d'électrolyte.

Parmi les sels les plus utilisés figure l'hexafluorophosphate de lithium (LiPF₆), qui possède beaucoup des nombreuses qualités requises mais présente le désavantage de se dégrader sous forme de gaz d'acide fluorhydrique par réaction avec l'eau. Cela pose des problèmes de sécurité, notamment dans le contexte de l'utilisation prochaine des batteries lithium-ion pour les véhicules particuliers.

Récemment, d'autres sels ont été développés, tels que le LiTDI (le 1-trifluorométhyl-4,5-dicarbonitrile-imidazolate de lithium) et le LiPDI (1-pentafluoroéthyl-4,5-dicarbonitrile-imidazolate de lithium). Ces sels présentent les avantages de posséder moins d'atomes de fluor et d'avoir des liaisons fortes carbone-fluor en lieu et place des liaisons plus faibles phosphore-fluor du LiPF₆.

Par ailleurs, le brevet WO2010023413 montre que ces sels présentent des conductivités de l'ordre de 6 mS/cm, une très bonne dissociation entre l'anion imidazolate et le cation lithium et leur utilisation en tant que sel d'électrolyte de batteries Li-ion. Mais cette conductivité ionique mesurée dans les solvants dits classiques des électrolytes que sont les mélanges de carbonates est trop faible pour une utilisation dans les batteries dites de type puissance.

Le demandeur a découvert que l'utilisation de solvant présentant au moins une fonction nitrile permet d'améliorer la conductivité ionique de ces sels de lithium.

### DESCRIPTION

L'invention concerne en premier lieu une composition électrolytique comprenant un ou plusieurs sels de lithium de formule (A) dans un solvant comportant au moins une fonction nitrile ou un mélange de solvants dont au moins un comporte une fonction nitrile.

L'invention a également pour objet l'utilisation dudit électrolyte comme électrolyte de batteries Li-ion.

L'invention a en outre pour objet l'utilisation d'un solvant comportant au moins une fonction nitrile pour améliorer la conductivité ionique d'électrolyte à base de sels d'imidazolate de lithium.

Les sels de lithium de formule (A) sont représentés ci-dessous 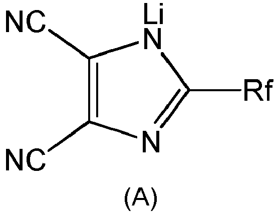 avec Rf représentant un atome de fluor, un groupement nitrile, un groupement alkyle éventuellement fluoré ou perfluoré ayant de 1 et 5 carbones, un groupement alcoxy éventuellement fluoré ou perfluoré ayant de 1 à 5 carbones ou un oxa-alcoxy éventuellement fluoré ou perfluoré ayant 1 à 5 carbones.

A titre d'exemples, on peut notamment citer les groupes Rf suivants : F, CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F₇, C₄H₄F₅, C₅F₁₁, C₃F₅OCF₃, C₂F₄OCF₃, C₂H₂F₂OCF₃, CF₂OCF₃, C₅F₁₁OCH₃, CF₂OC₂H₅, CF₂OC₂H₄OCH₃, CF₂OC₂H₄OC₂H₅, CF₂OCH₂OCF₃, CF(CF₃)OCH₃, CF(CF₃)OC₂H₅, CF(CF₃)OC₂H₄OCH₃ ou CF(CF₃)OC₂H₂F₃.

De préférence, Rf représente CF₃ .

La présente invention permet de surmonter les inconvénients des sels précédemment décrits. En effet, bien que ces sels soient particulièrement intéressants du fait de leur stabilité chimique et électrochimique et de la forte séparation entre l'anion et le cation lithium, toutefois leur faible conductivité ionique limite leur performance en terme de puissance. Cette faible conductivité peut être attribuée non seulement à une faible dissociation du sel dans les solvants classiques d'électrolyte, notamment les carbonates, mais aussi à une viscosité élevée due à la taille de l'anion. Comme carbonates classiquement utilisés, on peut citer notamment l'éthylène carbonate, le diméthylcarbonate, l'éthylméthylcarbonate, le diéthylcarbonate, le propylène carbonate.

Le demandeur a découvert que l'utilisation de solvant comportant au moins une fonction nitrile en présence ou non de co-solvant(s) permet d'améliorer grandement la conductivité ionique des électrolytes faits à partir des sels de lithium de formule (A). Sans être lié par une quelconque explication, le demandeur pense que la fonction nitrile de part sa planéité et de son affinité avec les fonctions nitriles de l'anion du sel de formule (A) permet une meilleure dissociation du sel de lithium De plus, les solvants comportant au moins une fonction nitrile possèdent des avantages comparés aux solvants classiques. Ainsi, la faible viscosité et la large gamme de température à laquelle les solvants comportant au moins une fonction nitrile sont à l'état liquide offrent une gamme de température de fonctionnement plus étendue de la batterie.

Les solvants comportant au moins une fonction nitrile selon la présente invention peuvent être représentés par la formule générale R(CN)ₓ où x est un nombre compris entre 1 et 3 et R représente un groupement alkyle éventuellement fluoré ou perfluoré ayant de 1 et 5 carbones, un groupement alcoxy éventuellement fluoré ou perfluoré ayant de 1 à 5 carbones ou un oxa-alcoxy éventuellement fluoré ou perfluoré ayant 1 à 5 carbones, de préférence, les solvants étant aprotiques comme le propionitrile.

Selon un mode de réalisation x est égal à 2 et R a la même signification que ci-dessus. Le glutaronitrile, le méthoxyglutaronitrile, le 2-méthylglutaronitrile, le 3-méthylglutaronitrile, l'adiponitrile et le malononitrile sont préférés.

Comme solvants comportant au moins une fonction nitrile, on peut citer notamment l'acétonitrile, le pyruvonitrile, le propionitrile, le méthoxypropionitrile, le diméthylaminopropionitrile, le butyronitrile, l'isobutyronitrile, le valéronitrile, le pivalonitrile, l'isovaléronitrile, le glutaronitrile, le méthoxyglutaronitrile, le 2-méthylglutaronitrile, le 3-méthylglutaronitrile, l'adiponitrile et le malononitrile.

Le solvant nitrile peut être utilisé seul ou en mélange avec un ou cinq co-solvants.

Comme co-solvants, on peut citer notamment les nitriles de formule R(CN)ₓ précédemment cités, les carbonates tels que l'éthylène carbonate, le diméthylcarbonate, l'éthylméthylcarbonate, le diéthylcarbonate, le propylène carbonate ou les glymes tels que l'éthylène glycol diméthyléther, le diéthylène glycol diméthyléther, le dipropylène glycol diméthyléther, le diéthylène glycol diéthyléther, le triéthylène glycol diméthyléther, le diéthylène glycol dibutyléther, le tétraéthylène glycol diméthyléther et le diéthylène glycol t-buthylméthyléther.

De préférence, le(s) solvant(s) comportant au moins une fonction nitrile représente(nt) entre 1% et 100% en volume de la totalité des solvants dans la composition électrolytique, avantageusement entre 10 et 90% en volume.

Plus particulièrement, en présence de solvants de formule R(CN)ₓ dans laquelle x est égale à 2 ou 3, le ou les co-solvants est ou sont, de préférence, choisi(s) parmi le diméthylcarbonate, l'éthylméthylcarbonate, le diéthylcarbonate, le propylène carbonate, l'éthylène glycol diméthyléther, le diéthylène glycol diméthyléther, le dipropylène glycol diméthyléther, le diéthylène glycol diéthyléther et le triéthylène glycol diméthyléther.

Selon ce mode de réalisation (c-à-d lorsque x = 2 ou 3), la proportion en volume de solvant(s) comportant au moins une fonction nitrile R(CN)ₓ dans le mélange de solvants est, de préférence, comprise entre 1% et 50% et la proportion en volume de la somme des co-solvants est, de préférence, comprise entre 50% et 99% du volume total du mélange.

Selon un autre mode de réalisation, en présence de solvants de formule R(CN)ₓ dans laquelle x est égale à 1, le ou les co-solvants est ou sont, de préférence choisi(s) parmi l'éthylène carbonate, le propylène carbonate, le diéthylène glycol dibutyléther, le tétraéthylène glycol diméthyléther et le diéthylène glycol t-buthylméthyléther.

Selon ce mode de réalisation (c-à-d lorsque x = 1), la proportion en volume de solvant(s) comportant au moins une fonction nitrile R(CN)ₓ dans le mélange de solvants est, de préférence, comprise entre 50 et 99 % et la proportion en volume de la somme des co-solvants est, de préférence, comprise entre 1% et 50% du volume total du mélange.

La quantité de sel de lithium de formule (A) dissous dans le mélange de solvants décrits ci-dessus peut varier entre 0,01 et 10 mol/l, plus préférentiellement entre 0,05 et 2 mol/l.

La quantité de sel de lithium de formule (A) présente dans la composition électrolytique selon la présente invention peut varier entre 0,01 et 10 mol/l, de préférence entre 0,05 et 2 mol/l.

De préférence, le(s) sel(s) de lithium de formule (A) représente(nt) entre 2% et 100% en poids de la totalité des sels présents dans la composition électrolytique, avantageusement entre 25 et 100% en poids.

La présente invention a également pour objet l'utilisation d'au moins un un solvant comportant au moins une fonction nitrile pour améliorer la conductivité ionique d'électrolyte à base de sels d'imidazolate de lithium, de préférence de formule (A).

La quantité de solvant comportant au moins une fonction nitrile mise en jeu est de préférence celle indiquée ci-dessus.

Le solvant nitrile est de préférence choisi dans la liste décrite précédemment.

La présente invention a en outre pour objet l'utilisation des compositions précitées comme électrolyte de batteries Li-ion.

### EXEMPLES

Les exemples suivants illustrent l'invention sans la limiter. Dans les exemples suivants les conductivités ioniques ont été mesurées par spectroscopie d'impédance à l'aide de cellule de conductivité munie de 2 plaques en platine platinée.

### Exemple 1 :

Un électrolyte contenant 1 mol/l d'un sel de formule (A) où Rf = CF₃ (LiTDI) dans le propionitrile est préparé. La conductivité ionique de cet électrolyte est mesurée à l'aide de la technique précédemment décrite. La valeur obtenue est de 12 mS/cm.

### Exemple 2 :

Un électrolyte contenant 0,9 mol/L d'un sel de formule (A) où Rf = CF₃ (LiTDI) dans un mélange éthylène carbonate / propionitrile avec différentes proportions en volume est préparé. Les conductivités ioniques de ces électrolytes sont mesurées à l'aide de la technique précédemment décrite. Les valeurs obtenues sont résumé dans le tableau suivant.

| **Rapport en volume EC/propionitrile** | **Conductivité ionique** |
|---|---|
| 10/90 | 10,44 |
| 20/80 | 9,91 |
| 30/70 | 9,25 |
| 40/60 | 8,54 |
| 50/50 | 7,76 |

## Revendications

1. Composition électrolytique comprenant au moins un sel de lithium de formule (A) 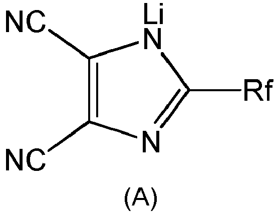 dans laquelle Rf représente un atome de fluor, un groupement nitrile, un groupement alkyle éventuellement fluoré ou perfluoré ayant de 1 et 5 carbones, un groupement alcoxy éventuellement fluoré ou perfluoré ayant de 1 à 5 carbones ou un oxa-alcoxy éventuellement fluoré ou perfluoré ayant 1 à 5 carbones,
dans un solvant comportant au moins une fonction nitrile ou un mélange de solvants dont au moins un comporte une fonction nitrile.

2. Composition selon la revendication 1 **caractérisée en ce que** le solvant comportant au moins une fonction nitrile est représenté par la formule générale R(CN)ₓ où x est un nombre compris entre 1 et 3 et R représente un groupement alkyle éventuellement fluoré ou perfluoré ayant de 1 et 5 carbones, un groupement alcoxy éventuellement fluoré ou perfluoré ayant de 1 à 5 carbones ou un oxa-alcoxy éventuellement fluoré ou perfluoré ayant 1 à 5 carbones, de préférence un solvant étant aprotique.

3. Composition selon la revendication 1 ou 2 **caractérisée en ce que** Rf représente F, CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F₇, C₄H₄F₅, C₅F₁₁, C₃F₅OCF₃, C₂F₄OCF₃, C₂H₂F₂OCF₃, CF₂OCF₃, C₅F₁₁OCH₃, CF₂OC₂H₅, CF₂OC₂H₄OCH₃, CF₂OC₂H₄OC₂H₅, CF₂OCH₂OCF₃, CF(CF₃)OCH₃, CF(CF₃)OC₂H₅, CF(CF₃)OC₂H₄OCH₃ ou CF(CF₃)OC₂H₂F₃.

4. Composition selon la revendication 3 **caractérisée en ce que** Rf représente CF₃

5. Composition selon l'une quelconque des revendications précédentes **caractérisée en ce que** la quantité de sel de lithium de formule (A) présente dans la composition électrolytique selon la présente invention peut varier entre 0,01 et 10 mol/l, de préférence entre 0,05 et 2 mol/l.

6. Composition selon l'une quelconque des revendications précédentes **caractérisée en ce que** le(s) solvant(s) comportant au moins une fonction nitrile représente(nt) entre 1% et 100% en volume de la totalité des solvants dans la composition électrolytique.

7. Composition selon l'une quelconque des revendications 2 à 6 **caractérisée en ce que** x est égal à 2.

8. Utilisation d'une composition selon l'une quelconque des revendications précédentes dans une batterie Li-on.

9. Utilisation d'un solvant comportant au moins une fonction nitrile ou un mélange de solvants dont au moins un comporte une fonction nitrile pour améliorer la conductivité ionique d'électrolyte à base de sels d'imidazolate de lithium, de préférence de formule (A).

10. Utilisation selon la revendication 9 **caractérisée en ce que** le sovant nitrile est représenté par la formule générale R(CN)ₓ où x est un nombre compris entre 1 et 3 et R représente un groupement alkyle éventuellement fluoré ou perfluoré ayant de 1 et 5 carbones, un groupement alcoxy éventuellement fluoré ou perfluoré ayant de 1 à 5 carbones ou un oxa-alcoxy éventuellement fluoré ou perfluoré ayant 1 à 5 carbones, de préférence aprotique.

11. Utilisation selon la revendication 9 ou 10 **caractérisée en ce que** x est égal à 2.

12. Utilisation selon l'une des revendications 9 à 11 dans une batterie li-ion.
